**Europäisches Patentamt**

**European Patent Office**

**Office européen des brevets**

(19)

(11) **EP 1 302 866 A1**

(12) **EUROPEAN PATENT APPLICATION**

(43) Date of publication:
**16.04.2003 Bulletin 2003/16**

(51) Int Cl.$^7$: **G06F 17/30**, G06F 17/50

(21) Application number: **01402656.1**

(22) Date of filing: **15.10.2001**

| | |
|---|---|
| (84) Designated Contracting States:<br>**AT BE CH CY DE DK ES FI FR GB GR IE IT LI LU MC NL PT SE TR**<br>Designated Extension States:<br>**AL LT LV MK RO SI**<br><br>(71) Applicant: **Cerep SA**<br>**92506 Rueil Malmaison (FR)** | (72) Inventor: **Horvath, Dragos**<br>**59260 Hellemmes (FR)**<br><br>(74) Representative: **Becker, Philippe et al**<br>**Cabinet Becker & Associés**<br>**35, rue des Mathurins**<br>**75008 Paris (FR)** |

(54) **Method of predicting biological activity profiles based on the neighborhood behavior of "in silico" similarity metrics calibrated with respect to activity spaces**

(57)     The present invention uses the analysis of the experimental responses of compounds with respect to a panel of multiple activity tests to optimize the Neighborhood Behavior of molecular similarity metrics in a Structural Space (the assumption that structurally similar compounds display similar biological properties), and subsequently applies the herein established relationship between the structural space and the activity test panel in order to predict the most likely activity profiles of a novel compound on the basis of the (experimentally measured) ones of its nearest neighbors among a reference set of drugs, druglike molecule and other chemical compounds. The dual nature of the Neighborhood Behavior is described in terms of complementary aspects following complementary quality criteria to calibrate and define the proficiency of In Silico structural space to map on the Activity Space.

**EP 1 302 866 A1**

**Description**

The invention relates to a method of computational prediction of the most likely biological activity profiles of compound sets against a large panel of targets covering different therapeutic areas, using the neighborhood behavior of specifically calibrated "In Silico" molecular similarity metrics with respect to biological activity spaces.

As a consequence of advances in the field of High Throughput Screening (HTS), the systematic testing of compounds against an entire panel of targets coves different therapeutic areas, defining their *"In Vitro* profile". This testing is nowadays used to generate a wealth of relevant information with respect to the expected *In Vivo* behavior of such drug candidates.

Profiling or High Throughput Profiling (HTP) generates a consistent set (vector) of experimental activities characterizing the behavior of the compound with respect to the considered panel of targets. The compound can therefore be assimilated to a well-defined point in a multidimensional "Activity Space" (AS) in which every axis monitors the response in one of the biological tests in the panel. However, the development of chemo-informatics tools required for the analysis and prediction of such profile data remains in an incipient phase. Consequently, molecular structures are represented, in a Structural Space (hereafter so-called "SS") by points defined by calculated vectors of molecular descriptors. The distances between such points are given by a molecular dissimilarity metric of that SS, as described for instance in the Journal of Chemistry Information and Computer Sciences, 1998, n° 38, pages 983-996.

In order to be relevant as a tool for biological properties prediction, a Structural Space should display a significant "Neighborhood Behavior" (further on the so-called NB) with respect to the experimental activity or property under study, according to the NB principle. Such a principle states that near neighbors in the structural space are statistically likely to display similar biological activity profiles (e.g. appear as near neighbors in the biological activity space).

For instance, a subset of structurally nearest neighbors of an active compound should expectedly include more actives than any other, randomly chosen, set of the same size. Neighborhood Behavior is the underlying working hypothesis in a whole series of time-and-expense-saving computational applications based on the molecular similarity concept, including: cluster analysis of a compound set in SS, the search for nearest neighbors of an active compound within a collection of yet untested, or even unsynthesized compounds ("virtual screening"). If a SS displays a strong NB, the properties of a given (untested or even virtual) molecule can be estimated as an average of the properties of its (experimentally characterized) nearest neighbors in SS, e.g. NB can be used for predictive purposes, in a series of approaches generally referred to as "K Nearest Neighbors" (KNN) methods.

The development and calibration of activity-relevant structural similarity metrics, an essential prerequisite for a successful computational exploitation of the "similarity hypothesis", requires a clear-cut and objective definition of NB. Up to date, see Journal of Medical Chemistry, 1998, n°41, pages 478-488, the NB of various metrics has been monitored with respect to a single molecular property at a time - the "main" activity of the compound in the therapeutic area it had been designed for.

By contrast to prior art, the Neighborhood Behavior according to the present invention is based on the exploitation of the information offered by the entire activity profile of the compounds against the representative panel of biological targets activity. Therefore it breaks the implicit assumption of the prior art that each compound is member of a single "major" activity class. Conversely, the herein described method can be used to estimate an entire activity profile - and not only a single molecular property - on the basis of the profiles of its nearest neighbors in SS, by means of an improved KNN-type method.

The criteria used in prior art to monitor the NB of various dissimilarity metrics - cluster separation analysis, population of activity classes in maximal diversity selections and analysis of the activity versus In Silico dissimilarity plots - do not integrate the concept of activity profile, but are based on more or less rigorously defined "activity classes" in terms of the supposedly "major" activity of the compound against the target it was "designed for".

Cluster separation analysis monitors the distribution of actives of different biological classes within each cluster of compounds in Structural Space, following the principle that binders to different biological targets should not cluster together. This is in principle a sound approach, but its dependence on the choice of the clustering procedure and its adjustable parameters introduces a supplementary level of empirism - sometimes it may be very difficult to decide where to draw the borderline between two "clusters". In particular, it is not always true that any pair of members of a same cluster is always situated closer together than they are with respect to members of different, neighboring clusters.

Monitoring of the number of activity classes sampled by a most diverse compound selection relies on the assumption that picking of compounds that are as far as possible from each other in Structure Space should avoid redundant sampling of a same activity class. This approach has been also used for similarity metric optimization.

However, it relies on the biased assumption that members of a same class have to be structurally related. A metric that succeeds in grouping together the members of an activity family into a minimal number of clusters might suffer from an overstatement of the similarity of members of actually different subfamilies within a same activity class.

In other words, imposing that the set of structurally dissimilar pairs should only include pairs of members of different activity classes is equivalent to requiring that all the compound pairs with similar activities should be structurally similar.

This is different from the more reasonable condition that compound pairs with similar activities should be the only ones scoring low structural dissimilarity.

The present invention relies on this latter condition to calibrate higher quality In Silico similarity scores in order to more accurately interpolate the biological activity profiles of said compounds on the basis of their nearest neighbors among an experimentally studied reference set.

It uses specifically calibrated "In Silico" molecular similarity metrics to retrieve the nearest neighbors of a predicted compound within a set of reference molecules of known biological activity profiles, and to estimate its properties on the basis of those of its neighbors, according to the NB principle. More specifically, the In Silico similarity metrics are calibrated in a structure space in order to maximize the validity of the "Neighborhood Behavior" principle, meaningful activity metrics being defined in order to estimate how dissimilar the biological responses of two compounds are in an activity space.

More precisely, the subject matter of the present invention is a method of predicting biological activity profiles of compounds against a(n entire) panel of targets covering different therapeutic areas. Experimental activity profiles define a multidimensional Activity Space. Chemical compounds are represented in the activity space by activity profile vectors given by their experimental responses in each of included tests, this activity space characterizing a molecule in terms of its pharmacological properties (including receptor binding properties, enzyme binding properties, behaviour in selected cellular tests). A compound location in the Activity Space is estimated by Neighborhood Behavior on the basis of its calculated location in a Structural Space that optimally maps onto the Activity Space. An optimal mapping is understood as considering that neighboring points in the former Space are likely to be neighbors in the latter Space. Dissimilarity activity metrics being applied to the Activity Space, the In Silico molecular dissimilarity metrics that apply to the Structure Space are calibrated in order to optimize said structure-to-activity mapping by achieving a best compromise between two quantitative NB criteria:

- "consistency", the ability of the In Silico metric to specifically avoid predicting as similar pairs of compounds with different activities, and

- "completeness", its ability to retrieve a maximum of similar compounds pairs with similar activity profiles among those present in the data set.

This method specifically defines Neighborhood Behavior in terms of the complementary aspects of "consistency" and "completeness" embodied by quantitative definitions of the proficiency of In Silico Structural Space to map on the Activity Space. Molecules represented by neighboring points in the Structural Space display then a higher-than-random probability to be neighbors in Activity Space as well - e.g. will display related activity profiles.

Furthermore, NB criteria are advantageously used as objective scores for the choice of the optimal functional form and fittable parameters of In Silico metrics based on Fuzzy Bipolar Pharmacophore Autocorrelograms (further on FBPA). A validation assessing the NB quality of the optimal FBPA metrics against different compounds in a completely different activity space is considered in order to cross-check the extent to which the optimal parameters of this metric are biased by the actual choice of targets in the panel used for calibration.

In particular, the use of customized activity profiles serves to define "privileged" activity spaces for the training of "focused" similarity metrics with a maximal discriminative power with respect to characteristic ligands for the classes of receptors entered in the profile. However, the testing of a similarity metric calibrated on the basis of an activity panel dominated by G-Protein Coupled Receptors (further on "GPCR") against a completely different activity space establishes that, in spite of the initial bias in terms of targets, the fitting of pharmacophoric weighing factors defines a metric maintaining its NB properties outside the activity space it had been conceived for.

After calibration and validation of the In Silico similarity metrics, the method of prediction according to the invention can be applied to predict an unknown biological property (e. g. binding affinities, ADME/Toxicity related features) of an untested, or even unsynthesized, molecule M, when a calibrated metric of optimal NB is given as defined above. The activity profile of M will be estimated as a weighted average interpolation of the corresponding property profiles of its nearest neighbors $m_1, m_2,...,m_K$, from a reference set of compounds for which the measurement of the properties of interest had been previously undertaken.

By contrast to prior approaches, - for example, the method of "K Nearest Neighbors" (KNN) predicting the property of a novel compound to equal the arithmetic average of the properties of its K nearest neighbors, with K being a fixed number (typically 5) -, the present approach can also introduce:

- a variable number of considered near neighbors: all reference compounds within a given dissimilarity radius, $\Delta S$ $(m,M)<\delta$, are used in calculation;

- a weighted averaging of the neighbor properties, with closer neighbors having larger weights; the weight $W(m)$ of each neighbor "m" is assumed to decrease exponentially with the considered In Silico dissimilarity score;
- confidence descriptors, critically assessing whether the use of the model is relevant to a given compound, based on:
- an "Effective Number of Neighbors" of M defined as $\Sigma W(m)$ (e.g. ,if there are K reference molecules m of negligible dissimilarity $\Delta S(M,m)\approx 0$, then the effective number of neighbors of M would equal K);
- the spread of experimental values of the near neighbors: if NB is locally applicable, then all the neighbors of M should have closely related $P(m)$ values, for they also are neighbors of each other. Therefore, the effective weighted spread of the $P(m)$ values $RMS_W=sqrt[\ (<P(m)>|_W)^2 - <P(m)>^2|_W]$ is a measure of homogeneity in the neighborhood of M, $RMS_w=0$ meaning maximal homogeneity, whereas $RMS_W$ equaling the spread $RMS_P$ of the property P over the entire reference set means that NB-based prediction of $P(M)$ would be completely irrelevant; and
- an overall confidence score is defined as the product of EFN and $(RMS_P-RMS_W)/RMS_P$, with the property of being maximal when M has many close neighbors and all these neighbors consistently display close values of the property P. The confidence score value is systematically output next to the predicted $P(M)$, the predictive model being thus able to point out the limits of its own applicability.

The approach is interactive, allowing the users to customize the list of near neighbors "m" suggested by the similarity metric, discarding those that should be considered irrelevant.

Thus, the method of prediction according to the invention can use more particularly:

- a variable number of all the near neighbors within a given dissimilarity radius $\Delta S(m,M)<\delta$ and a weighted averaging of the neighbor properties, with closer neighbors having larger weights, based on the NB principle, the weight $W(m)$ of each neighbor m is assumed to decrease exponentially with the considered In Silico dissimilarity score $\Delta S(m,M)$;

- the "Effective Number of Neighbors" of the molecule M defined as $\Sigma W(m)$, the neighbors that are further away from the molecule M counting less than one "effective" neighbor;

- the effective weighted spread of the $P(m)$ values, $RMS_w$, which is a measure of homogeneity in the neighborhood of M, expectedly lower than the overall spread $RMS_P$ of that property over the entire set of compounds, and

- the overall confident score is defined as the product of EFN and $((RMS_P-RMS_W)/RMS_P)$, such a confident score being maximal when the molecule M has many close neighbors and all these neighbors consistently displaying close values of the property P.

According to particular embodiments of the method of prediction according to the present invention :

- the In Vitro dissimilarity scores $DA(m,M)$ are defined as a fuzzy measure of the effective number of targets with respect to which two compounds display a significant difference in affinity, the said significant difference in activity being determined in function of the difference of the experimental percentages of inhibition achieved by m and M in that biological test, at a concentration of $10\mu M$; and

- the said effective number of targets is with respect to which two compounds display a significant difference in affinity is scaled on the basis of inter-target correlation coefficients $R(t,T)$ in order to account for the potential relatedness of different targets t and T included in the activity profile.

- a consistency criterion $\sigma$ captures the proficiency of a structural similarity metric to selectively perceive pairs of activity-related compounds as structurally similar and is defined as the average of the activity similarity score $<DA(m,M)>_{P(\delta)}$ over a pair subset $P(\delta)$ with structural dissimilarity lower than $\delta$, interpreted relative to the one achieved by a randomly picked subset of the same size $N_{P(\delta)}$ - an upper threshold value that can be ultimately assimilated to the global average over the entire set of pairs $<DA(m,M)>_P$ - and respectively a lower threshold $<DA(m,M)>_{O(\delta)}$, where $O(\delta)$ denotes a subset of the same size as $P(\delta)$, containing the $N_{P(\delta)}$ most similar pairs in activity space itself.

- an overall optimality criterion $\Omega$ is defined by comparing the weighted sum $\kappa N_{FS}+N_{PFD}$ of the number of $N_{FS}$ "falsely similar" pairs, -compound pairs with an In Silico dissimilarity below a given threshold $\delta$, but In Vitro activity dissimilarity above a given threshold d- , and the number of $N_{PFD}$ "potentially false dissimilar" pairs, i.e. compound pairs with an In Silico dissimilarity above $\delta$, but In Vitro activity dissimilarity below d, to the value of this sum that would have been achieved by a random selection of an equal size compound pair set instead of the selection based on the actual In Silico dissimilarity scores;

Other features and advantages of the present invention will be disclosed in the hereafter detailed description of non limitative embodiments in reference with the annexed figures which respectively show :

- Figure 1, pairwise correlations R(t,T) between the activities of the compound set against different targets ;

- Figure 2, criteria $\Omega$-$\sigma$ plot of the calibrated FBP0 metric working in the absolute space of pharmacophore type atom pair counts, against its FBP*0 using A/V normalized FBPA descriptors, at the same weighing and fuzziness factor setup, all the $\Omega$-$\sigma$ plots employing the AS metric $NR_{difW}$;

- Figure 3, Consistency within the set of 4500 pairs ranked as the most similar by various FBPA metrics, with respect to the $NR_{diff}$ activity metric;

- Figure 4, $\Omega$-$\sigma$ plots illustrating the NB of various FBPA similarity scoring formalisms at fixed weighing $w_k$ and fuzziness $\alpha$ factors;

- Figure 5, $\Omega$-$\sigma$ plots illustrating the impact of the choice of the pharmacophore feature weighing factors $w_k$ on the NB of FBPA similarity scores;

- Figure 6, Comparative $\Omega$-$\sigma$ plots of three metrics (not optimized, partially optimized and thoroughly optimized) within the validation environment; and

- Figure 7, Comparative $\Omega$-$\sigma$ plots of three FBPA metrics (not optimized, partially optimized and thoroughly optimized), monitoring their performances with respect to completely different training and validation environments.

The early assessment of potential liabilities of tens to hundreds of lead compounds represents the principal milestones of the path towards the drug candidate. It relies on robotized HTP, monitoring the activities $A_{ij}$ of compounds i (i=1...$N_{comp}$) against a series of j=1...$N_{assay}$ receptor and enzyme binding and/or ADMET (i.e. Absorption, Distribution, Metabolism, Excretion, and Toxicity) - related assays. The resulting experimental output - the "activity profiles" of compounds - provides an extensive characterization of *In Vitro* biological properties. In this context, a compound i is represented by an activity profile vector denoting a point $\vec{P}_i = (A_{ij})_{j=1,Nassay}$ in the Activity Space.

The objective evaluation of the NB of In Silico dissimilarity metrics according to the present invention is based on experimental activity profiles of compounds against multiple biological targets in the AS. Prior to the definition of the NB criteria, dissimilarity (distance) metrics need to be introduced both in the Activity Space ("In Vitro" dissimilarity metrics evaluating how different two measured biological activity profile vectors are) and in Structural Space ("In Silico" molecular dissimilarity metrics).

The properties of the AS, determining the optimal way to define the "In Vitro" Activity Dissimilarity metrics between two compounds m and M, DA(M,m), depend both on the nature of the measured response and the composition of the test panel.

Although HTP experiments may readily include cellular and/or ADMET-related assays, the test panel has been restricted to receptor binding and enzyme inhibition tests, best illustrating the recognition of ligands by macromolecular targets. A panel of $N_{assay}$=43 tests defines the considered AS.

A total of $N_{comp}$=584 commercially available drugs, druglike reference ligands and precursors commonly used in pharmaceutical industry have been screened against these tests. The average percentages of inhibition at a 10 $\mu$M concentration based on duplicate tests have been measured.

A meaningful definition of activity dissimilarity metrics DA(m,M) needs to consider the partial and somewhat noisy character of the affinity information provided by the inhibition percentages at a single concentration.

An activity (In Vitro) metric related to the number of targets with respect to which two compounds M and m display "significant potency differences" has been defined on the basis of the following rules: if the percentages of inhibition of m and M against a target j differ by more than a high threshold value H%, typically 70%, then this difference is considered to be "significant" and the count of is incremented by one unit. If they differ by less than a low threshold L%, e.g. 30%, this difference is most likely due to experimental noise and will be ignored.

In intermediate situations, a subunitary linearly interpolated increment will be applied:

$$NR_{diff}(m,M) = \sum_{i=1}^{N_{assay}} \Delta_j(m,M) \quad where \, \Delta_j(m,M) = \begin{cases} 1 & if \left| A_{M,j} - A_{m,j} \right| \geq H\% \\ 0 & if \left| A_{M,j} - A_{m,j} \right| \leq L\% \\ \dfrac{\left| A_{M,j} - A_{m,j} \right| - L\%}{H\% - L\%} & otherwise \end{cases} \quad 1)$$

If T and T' are related targets, then the ability of the In Silico metric to distinguish binders from nonbinders of T somehow *implies* its ability to do so with respect to T'. Its success with respect to the second target should not count, for it is the consequence of target interrelatedness and not a merit of the metric.

Target intercorrelations are accounted hereafter by means of weighing the distance contribution $\Delta_j(m,M)$ of the target "j" by a subunitary factor, unless target "j" is "orthogonal" to all the others in the test panel - e.g. if the activities of the molecules against j are not correlated with the ones against other targets. In order to do so, a measure of the target interrelatedness R(T,t) has been adopted as:

$$R(T,t) = \frac{2 \sum_{i=1}^{N_{compy}} A_{i,T} A_{i,t}}{\sum_{i=1}^{N_{comps}} A_{i,T}^2 + \sum_{i=1}^{N_{compdsy}} A_{i,t}^2} \quad 2)$$

The weight $\omega_j$ of the increment $\Delta_j(m,M)$ contributed by target j to the activity distance between M and m becomes:

$$\omega_j = 1 / \sum_{t=1}^{N_{assay}} R^*(j,t) \quad where \, R^*(j,t) = \begin{cases} R(j,t) & if \, R(j,t) \geq R_{min} \\ 0 & otherwise \end{cases} \quad 3)$$

where $\omega_j$=1 if R(j,t)<$R_{min}$ (typically 0.5) for any j≠t.

The introduction of a "minimal relevant correlation coefficient" $R_{min}$ was necessary in order to focus on *meaningful* target correlations only, avoiding possible artifacts due spurious summation of the numerous very small but positive R values of actually uncorrelated target pairs. It is now straightforward to define the "correlation-corrected" $NR_{difW}$ metric:

$$NR_{difW}(m,M) = \sum_{i=1}^{N_{assay}} \omega_j \Delta_j(m,M) \quad where \, \Delta_j(m,M) \, is \, defined \, in \, (6) \quad 4)$$

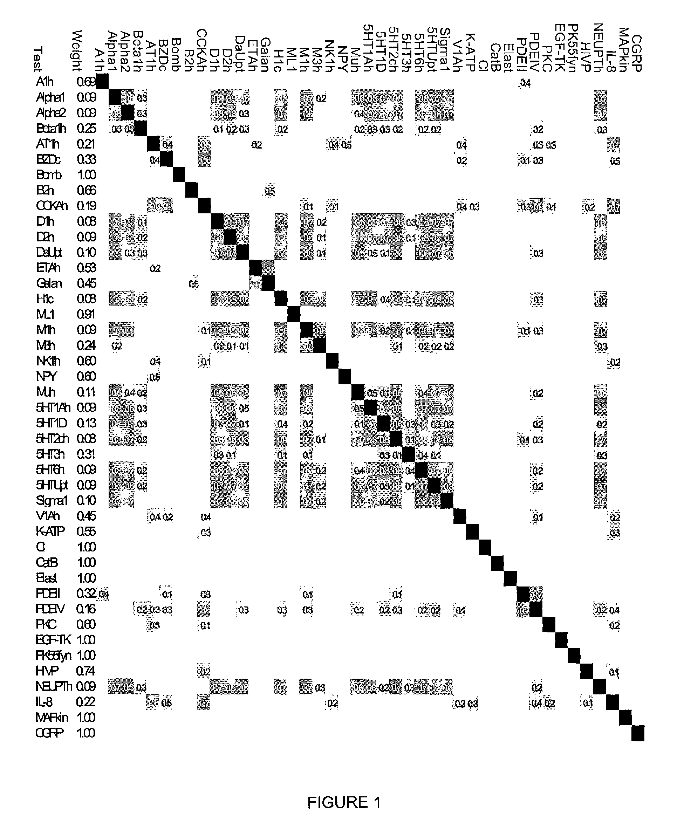

Figure 1 displays the correlation coefficients R(t,T) of the ligand responses against different targets t and T. The strongest correlations can be evidenced between biologically related members of the GPCR superfamily. Many of the found correlations merely reflect the phylogenetic relatedness (sequence homology) of those receptors. The strong bias in favor of GPCR targets in the activity profile and the evidenced correlations within this subset of targets induce the use of the "correlation-corrected" $NR_{difW}$ metric in the activity space.

Targets that appear to be completely uncorrelated to the other receptors of the panel are often found to display very low hit rates. For each GPCR receptors, the learning set includes at least 35 compounds scoring inhibition percentages above 50%, e.g. 5HT3h is the less promiscuous GPCR receptor, with 35 "hits" in the above-defined sense, while 5HT1A, 5HT2ch or Sigma1 have more than 160. By contrast, there are no significant "hits" for CGRP, IL-8, Elast, Cat-B and B2h and less than 5 hits for AT1h, Bomb, CCKAh, ETAh, Galan, NPY, NK1h, V1Ah, PKC, EGF-TK, Mapkin: the compound set under study, representative of the ensemble of the currently marketed drugs, is as well biased in favor of GPCR binders, GPCRs being both well characterized, important biological targets and yield acceptable hit rates in HTS experiments, which makes them attractive therapeutic targets.

The hereafter properties of the AS regards quantifying the similarity of activity profiles in a context where the measured activities $A_{ij}$ are expressed as percentages of inhibition at a 10 μM concentration, taking into account the potential bias due to the non-orthogonality of the axes defining the AS, -due to the relatedness of some receptors in the test panel-, on the AS similarity metrics. Further on, a generic symbol DA(m,M) will be used to design the activity dissimilarity score between two molecules M and m, in any context where the exact way in which this dissimilarity has been estimated is irrelevant.

Molecular structures, denoted by M and m, are represented by "points" of a Structural Space defined by their calculated vectors of molecular descriptors, in which the In Silico dissimilarity metric $\Delta S(m,M)$ represents the calculated "distance" between points.

In the current embodiment, the 252-dimensional Structural Space employed is defined in terms of known FBPA. However, the herein described methodology can be also applied with any other type of molecular descriptors and dissimilarity scores.

The FBPA of a molecule M, further on denoted as $\Psi_M(a,b,\Delta)$, monitors the numbers of atom pairs within each of the 252=21x12 categories that can be defined in terms of the 21 combinations of six pharmacophoric features, i.e. $a,b \in$ {Hydrophobicity, Aromaticity, Hydrogen Bond Acceptor & Donor, Cation, Anion} times 12 considered distance ranges $\Delta \in$ {(3..4), (4..5), ..., (14..15)} [Å]. By default, FBPA metrics were designed to work with the absolute number of atom pairs $\Psi_M(a,b,\Delta)$.

The FBPA space is homogeneous in the sense that all its axes monitor atom pair numbers of given pharmacophoric properties at given separation distance ranges. Nevertheless, the pharmacophoric feature pairs are unequally represented within the subset of pharmacologically relevant compounds. While aromatic and aliphatic groups are ubiquitous, charged features are relatively rare.

In the context of the "privileged" structural subspace populated by biologically active molecules, Average/ Variance (A/V) normalized FBPA show whether a compound contains more or less pairs than "expected" for an "average" drug molecule, in terms of "natural" units of observed pair number variances.

In Silico metrics explored hereafter are defined in following table 1 and discussed in the following paragraphs:

Table 1

| Metric | Definition | Description |
|---|---|---|
| FBP*.fd.optw | $\Delta FBP^{FD}$ | [0,2] - Average/Variance normalized FBPA metrics |
| FBP*.fd.optw2 | | using best 2 (sub)optimal parameterization schemes |
| FBP*.fd.eqw | $\Delta FBP^{FD}$ | [0,2] - Normalized FBPA with equal weights $w_k=1$ and "fuzziness" factor $\alpha=0.32$ |
| FBP*.eqw | $\Delta FBP^0$ | [0,2] - Normalized FBPA with $w_k=1$ and $\alpha=0.32$ |
| FBP*.d | $\Delta FBP^D$ | [0,2] - Dice metric with normalized FBPA |
| FBP*0 | $\Delta FBP^0$ | [0,2] - Normalized FBPA with "standard" parameters |
| FBP0 | $\Delta FBP^0$ | [0,1] - "Standard" FBPA metric (ref) |

- The original fuzzy-logic based similarity metric $\Delta FBP0(m,M)$ relies on partial similarity scores calculated with respect to the atom pair distributions matching each pharmacophore feature pair a,b:

$$\delta_{a,b}(m,M)=1-\frac{2(\Psi_m \otimes \Psi_M)_{a,b}}{(\Psi_M \otimes \Psi_M)_{a,b}+(\Psi_m \otimes \Psi_m)_{a,b}} \qquad 5)$$

where:

$$(\Psi_m \otimes \Psi_M)_{a,b}=\sum_{\Delta_1=1}^{N_{bin}}\sum_{\Delta_2=1}^{N_{bin}}\Psi_m(a,b,\Delta_1)\Psi_M(a,b,\Delta_2)exp\left[-\alpha(\Delta_1-\Delta_2)^2\right] \qquad 6)$$

- The global dissimilarity score is defined as a weighted average of the pharmacophore pair partial scores, with weighing factors $w_k$ associated to each pharmacophore feature:

$$\Delta FBP^0(m,M)=\frac{\sum_{a=1}^{N_f}\sum_{b=a}^{N_f}w_a w_b \delta_{a,b}(m,M)\Big|_{(\Psi_M \otimes \Psi_M)_{a,b}+(\Psi_m \otimes \Psi_m)_{a,b}>0}}{\sum_{a=1}^{N_f}\sum_{b=a}^{N_f}w_a w_b \Big|_{(\Psi_M \otimes \Psi_M)_{a,b}+(\Psi_m \otimes \Psi_m)_{a,b}>0}} \qquad 7)$$

- The fuzzy and weighted Dice metric, bypassing partial similarity score evaluation:

$$\Delta FBP^{FD}(m,M)=1-\frac{2\sum_{a,b}w_a w_b(\Psi_m \otimes \Psi_M)_{a,b}}{\sum_{a,b}w_a w_b(\Psi_M \otimes \Psi_M)_{a,b}+\sum_{a,b}w_a w_b(\Psi_m \otimes \Psi_m)_{a,b}} \qquad 8)$$

- The standard Dice metric - a particular case of equation 8) with weighing factors set to 1 and an infinitely large "fuzziness" parameter $\alpha$ from eq. 9):

$$\Delta FBP^D(m,M)=1-\frac{2\sum_{a,b}\sum_{\Delta}\Psi_m(a,b,\Delta)\Psi_M(a,b,\Delta)}{\sum_{a,b}\sum_{\Delta}\Psi_m(a,b,\Delta)\Psi_m(a,b,\Delta)+\sum_{a,b}\sum_{\Delta}\Psi_M(a,b,\Delta)\Psi_M(a,b,\Delta)} \qquad 9)$$

Dice similarity scores are sensitive to both the translation of the origin of the reference system and the rescaling of vector components. Average-Variance (A/V) normalization of the descriptors defining the structural space consists in choosing the average value of each descriptor over the entire compound set as the origin of the corresponding axis, and its variance as the "natural" unit, replacing the arbitrary unit in which the actual molecular descriptor had been expressed. This procedure is mandatory when using a heterogeneous selection of different categories of descriptors covering value ranges that are not directly comparable. The importance of A/V normalization with respect to the NB of *In Silico* metrics has been addressed, as detailed in Table 1.

Both the In Silico and activity dissimilarity scores have been generated according to different methods for the entire set of $N_{comp}$ x $(N_{comp}-1)/2 = 170236$ compound pairs, and used for the NB assessment. In the following, the notation $\Delta S(m,M)$ will be used to generically denote the In Silico dissimilarity score between two compounds m and M.

A first characteristic of a similarity metric is the pair density distribution: given a set P of $N_P$ compound pairs $(m,M) \in P$, let the distribution $\rho(P,d)$ represent the relative fraction of pairs $\delta N(d)/N_P$ with an activity dissimilarity comprised in a narrow range around a given value d. $\delta N(d)$ is the size of the subset $\{(m,M) \in P | |DA(m,M)-d|<\varepsilon\}$. By analogy, $\rho(P,\delta)$ denotes the pair density distribution of the In Silico metric. All the structural dissimilarity metrics $\Delta S$ considered are correlation coefficients that may take values within finite ranges $\Delta S_{min} \leq \delta \leq \Delta S_{max}$.

The present analysis of the NB of In Silico versus Activity Space metrics relies on molecular similarity understood in light of the following aspects for the development of NB scoring functions:

- high In Silico similarity, according to a valid metric, implies in a statistical sense an activity similarity of the concerned pairs: if $P(\delta)=\{(m,M)| \Delta S(m,M)<\delta\}$ is a subset of pairs that are structurally less dissimilar than $\delta$, then its activity similarity distribution $\rho(P(\delta),d)$ should be biased, in comparison to $\rho(P,d)$ over the entire set of pairs P, towards higher levels of population at lower d values; this bias increases with decreasing $\delta$;

- the reciprocal of the previous statement is not true, e.g. In Silico similarity is in no way *necessary* for activity similarity, in particular in the case of totally unrelated molecules that do not hit any of the targets and therefore display identical "blank" profiles. Structurally dissimilar compounds m and M may also share a common set of targets at which both bind, if (1°) m and M adopt different binding modes with respect to each target, or (2°) they have a same binding pharmacophore, but are nevertheless globally dissimilar because of structural differences that are neutral with respect to the binding properties, e.g. different moieties that protrude out of the site. In absence of knowledge about the binding process, overall In Silico similarity, accounting for all the features of a structure, is applied.

The consistency criterion captures the proficiency of a structural similarity metric to selectively perceive pairs of activity-related compounds as structurally similar. Accordingly, the average of the activity similarity score <DA(m,M)$>_{P(\delta)}$ over a pair subset $P(\delta)$ with structural dissimilarity lower than $\delta$ is a measure of consistency. This average is interpreted relative to the one achieved by a randomly picked subset of the same size $N_{P(\delta)}$ - an upper threshold value that can be ultimately assimilated to the global average over the entire set of pairs <DA(m,M)$>_P$. The lower threshold for <DA(m,M)$>_{P(\delta)}$ is <DA(m,M)$>_{O(\delta)}$, where $O(\delta)$ denotes a subset of the same size as $P(\delta)$, containing the $N_{P(\delta)}$ most similar pairs in activity space itself. $O(\delta)$ would be selected by an optimally discriminating In Silico metric able to rank the pairs in the natural order of their increasing activity dissimilarity. With these considerations, the consistency at a dissimilarity threshold $\delta$ can be written as:

$$\sigma(\delta) = \frac{\left\langle DA(M,m)\right\rangle_P - \left\langle DA(M,m)\right\rangle_{P(\delta)}}{\left\langle DA(M,m)\right\rangle_P - \left\langle DA(M,m)\right\rangle_{O(\delta)}} \qquad\qquad 10)$$

and may take values comprised between 1 (ideal NB) and 0 (random pair picking), or even below 0 if the In Silico "similarity" actively translates into activity dissimilarity.

The consistency criterion $\sigma$ implicitly monitors the "falsely similar" (FS) pairs for which the In Silico metric overemphasizes their actual structural similarity, causing them to be selected at $\Delta S<\delta$ in spite of their high activity dissimilarity. Compound pairs with high In Silico similarity scores, and high activity similarity values will be further on referred as "truly similar" (TS) pairs. Furthermore, the "truly dissimilar" (TD) pairs are defined as the In Silico dissimilar compound pairs also displaying significantly different activity profiles, whereas "potentially false dissimilar" (PFD) pairs have similar activity profiles, but are considered to be dissimilar by the In Silico metric. This denomination underlines that fact that not all the compound pairs with similar profiles and high In Silico dissimilarity are "falsely dissimilar", e. g. activity profile similarity by no means implies an expected low In Silico dissimilarity. However, in some cases the activity profiles similarity is an actual consequence of a "hidden" molecular similarity that is not reflected by the calculated In Silico dissimilarity score. The latter situations represent actual failures of the In Silico similarity metric that have to be taken into account when evaluating its overall quality.

The propensity of the In Silico metric to minimize the rate of "falsely dissimilar" pairs will be further on referred to as the "completeness" of the metric. A metric claiming that all but a few obviously related compound pairs are different (e.g. do not fall under the incidence of the "similarity hypothesis") may score an excellent consistency and yet be of limited usefulness, due to its failure to recognize the less obvious structural similarity relationships between other pairs of compounds with similar activity profiles.

If activity-related pairs are perceived as similar by at least one In Silico metric, then the ones failing to evidence this structural similarity (backed by activity similarity) need to be penalized. Activity-related pairs that are consistently seen as dissimilar by all In Silico metrics cannot be at this point "held against" the metrics, although some other metric able to evidence their "hidden" relatedness may nevertheless exist.

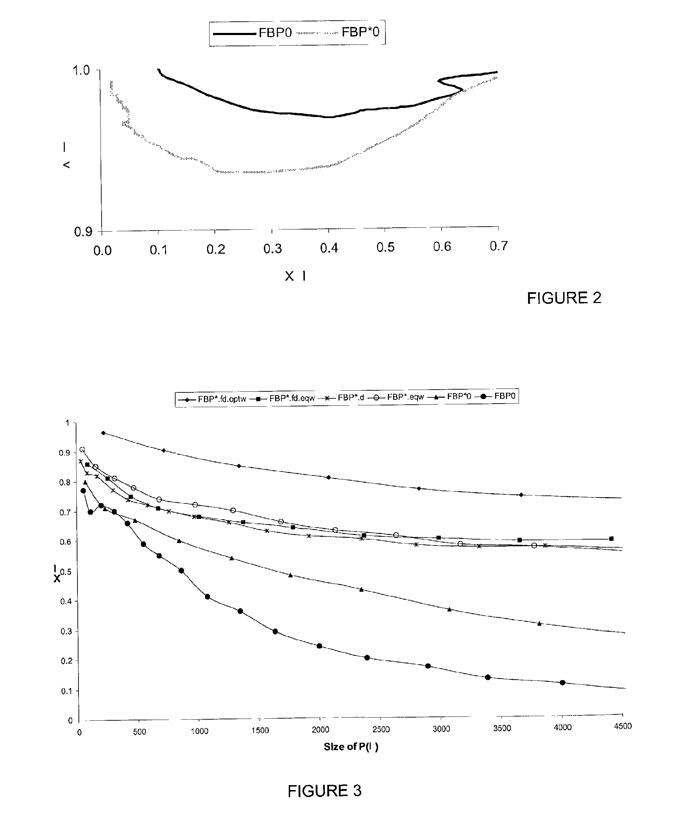

Plots of the consistency criterion $\sigma(\delta)$ against the *size* $N_{P(\delta)}$ of $P(\delta)$ (exemplified by Figure 3) offer at the same time an indirect illustration of completeness: a rapid decrease of $\sigma$ with respect to the set size suggests that the concerned metric may only achieve a high "consistency" only within a limited subset of few nearest neighbors, e.g. at low "completeness".

In order to derive a criterion that accounts for both consistency and completeness of a similarity metric, a double splitting of the pair set according to both an activity and a structural dissimilarity threshold, -d and $\delta$, respectively -, is shown in table 2 as follows:

| "Truly Similar" pairs: $N_{TS}$ | "Falsely Similar": $N_{FS}$ |
|---|---|
| DA(m,M)$\leq$d & $\Delta$S(m,M)$\leq\delta$ | DA(m,M)>d & $\Delta$S(m,M)$\leq\delta$ |
| "Potentially False Dissimilar" pairs: $N_{PFD}$ | "Truly Dissimilar": $N_{TD}$ |
| $N_{PFD}=N_{FD}+N^*_{TD}$ DA(m,M)$\leq$d & $\Delta$S(m,M)>$\delta$ | DA(m,M)>d & $\Delta$S(m,M)>$\delta$ |

Here, $N_{TS}+N_{PFD}$ is the number of pairs closer than d in Activity Space, representing a fraction $\alpha_d$ of the total pair number $N_p$. Similarly, the $N_{TS}+N_{FS}$ near neighbors pairs within a range $\delta$ of structural dissimilarity make out a fraction $\beta_\delta$ of the entire pair set. An In Silico metric with significant NB minimizes both the $N_{FS}$ and $N_{TFD}$ terms. Ideally, $N_{FS}=0$, while $N_{TFD}$ cannot decrease beyond the number $N^*_{TD}$ of "truly (structurally) dissimilar" pairs with similar activity profiles.

Since in practice the retrieval of false positives engenders losses in terms of unnecessary synthesis and testing efforts, the penalty related to $N_{FS}$ should be emphasized. Therefore, a scaled sum $\kappa N_{FS}+N_{TFD}$ may capture well the optimality requirements of a similarity metric, $\kappa$ being for instance set to 5. However, this sum depends on $\beta_\delta$ and is put in the context of the equivalent score achieved by a biologically irrelevant, random metric picking the same fraction $\beta_\delta$ of "similar" pairs. The fraction of activity-related pairs within the subset of randomly picked "structural neighbors" is equal to their overall occurrence $\alpha_d$, e.g. $N_{FS}^{(rand)}=\beta_\delta(1-\alpha_d)\times N_p$ and $N_{PFD}^{(rand)}=(1-\beta_\delta)\alpha_d\times N_p$. Since $\alpha_d$ is an intrinsic property of the learning set at a given d and $\beta_\delta=(N_{TS}+N_{FS})/N_p$, an overall optimality factor $\Omega^d(\delta)$, taking into account the two complementary aspects of "consistency" and "completeness" is defined as:

$$\Omega^d(\delta) = \frac{\kappa N_{FS} + N_{PFD}}{\kappa N_{FS}^{(rand)} + N_{PFD}^{(rand)}} = \frac{\kappa N_{FS} + N_{PFD}}{\left[\kappa\beta_\delta + \alpha_d - (\kappa+1)\beta_\delta\alpha_d\right]N_p} \qquad 11)$$

The superscript "d" indicates the dependence of this score on the choice of the activity similarity cutoff. It is observed that this dependence is irrelevant as far as d is chosen such as to encompass a reasonable fraction of <30% of nearest neighbor pairs in activity space. In all the studies reported here, d has been chosen such as to ensure $\alpha_d=15\%$ with respect to the concerned activity dissimilarity metric.

Now, the overall optimally criterion - a balanced account of both the consistency and the completeness of NB - is considered against the consistency criterion $\sigma(\delta)$. The former criterion equals 1 - the "null" value corresponding to a random selection - at both extremes of the structural similarity range, $\Omega(\Delta S_{min})=\Omega(\Delta S_{max})=1$. A local minimum at an intermediate value $\delta^*$ - the structural dissimilarity threshold allowing the selection of a significant number of activity-related pairs, is expected without co-opting an overwhelmingly large number of "false similars".

Since $\sigma(\delta)$ is a decreasing function of $\delta$, $\Omega(\delta)=f(\sigma(\delta))$ also displays a local minimum at $\sigma(\delta^*)$, of depth $1-\Omega(\delta^*)$. The *lower*, - that is "better"-, the overall optimality values at *high* consistency scores, the better is the Neighborhood Behavior of the structural metric.

The "quality" of a metric is considered relative to its intended applications, in which the relative importance of consistency vs. completeness needs to be carefully considered: the $NR_{difW}$ metric is the AS metric of choice used to build the criteria $\Omega$-$\delta$ plots throughout the present invention.

Figure 2 shows criteria $\Omega$-$\sigma$ plot of the calibrated FBP0 metric working in the absolute space of pharmacophore type atom pair counts, against its FBP*0 using A/V normalized FBPA descriptors at a same weighing and fuzziness factor setup, shows that A/V normalization reduces the afflux of "falsely similar" pairs, and therefore allows a wider selection of "truly similars" at a given consistency level $\sigma(\delta^*)\approx0.4$. This is consistent with the slower decrease of the FBP*0 consistency vs. selected subset size in Figure 3, within the set of 4500 pairs ranked as the most similar by various FBPA metrics, with respect to a $NR_{diff}$ activity metric, as defined previously.

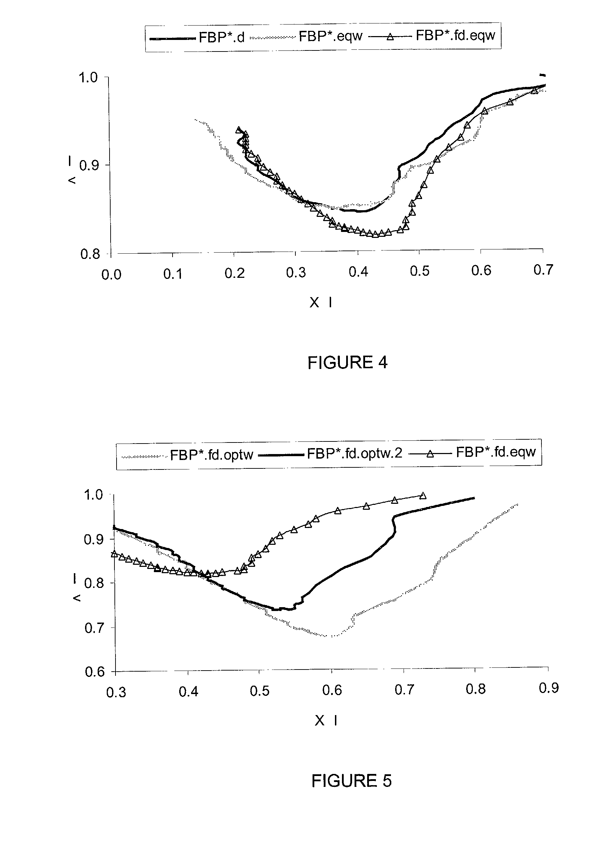

Figure 4 compares the relative performance of three different ways to estimate molecular dissimilarity scores from FBPA descriptors, at fixed configuration of the fittable parameters $w_k$=1, $\alpha$=0.32. The net increase in performance of the "fuzzy" FBP*.fd.eqw metric with respect to its "standard" Dice counterpart FBP*.d evidences the benefits of "blurring" the artificial cutoffs between the distance bins defining the FBPA. In consequence, the similarity score appropriately accounts for both the (main) contributions stemming from a same bin $\Psi(a,b,\Delta)$ being "populated", and the (smaller) contributions due to simultaneous population of different bins $\Psi(a,b,\Delta_1)$, $\Psi(a,b,\Delta_2)$ corresponding to close distance ranges. The "standard" scoring formula, here represented by the FBP*.eqw metric, appears to be less performant than the "fuzzy Dice" equation although it also benefits from "fuzzy logic" scoring.

An optimization of the seven fittable parameters $\Delta FBP^{FWD}$ metric, e.g. the six weighting factors $w_k$ associated to the pharmacophoric features and the fuzziness parameter $\alpha$, has been undertaken by means of a random search in a space defined by the limits 0<$w_k$<1 and 0.1<$\alpha$<1.5. At every explored configuration of parameters, the distance matrix $\Delta FBP^{FWD}(m,M)$ was rebuilt on the basis of the compound FBPA, and used to determine the consistency with respect to the $NR_{difW}$ activity metric within the set of 2000 most similar In Silico pairs. The FBP*.fd.optw metric is based on the herein determined optimal parameter configuration.

$$\sigma^{NRdifW}(\Delta FBP^{FWD})|_{NP(\Delta FBP)=2000} = MAX \qquad (12)$$

Figure 5 displays the outstanding improvement of NB obtained by calibration of the fittable parameters ($w_k$, $\alpha$). Optimizing the consistency of the metric with respect to the best 2000 most similar In Silico pairs, the resulting metrics spontaneously reached an excellent tradeoff between consistency and completeness.

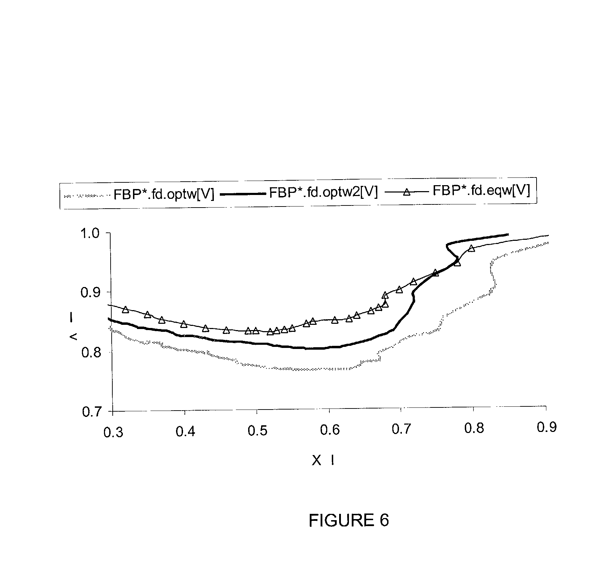

The relevance of the optimized metrics in a completely different training and validation environments than the one they were trained against is now checked. The NB validation test involved an Activity Space featuring a series of 12 novel targets that are not biologically related to any ones from the training set and a set 357 compounds, none of which was part of the initial molecule selection. The comparison of the performance of the three metrics in the validation environment showed in Figure 6 the same trend as evidenced in the training environment in Figure 5: the optimization procedure did not generate an overtrained metric, meaningless outside the environment it was developed for. The accentuation of the relative importance of aromatic and cationic features appears to be a generally favorable move towards improved NB, the relevance of these groups extending to other target families than GPCRs, cytochrome 2D6 being such an example.

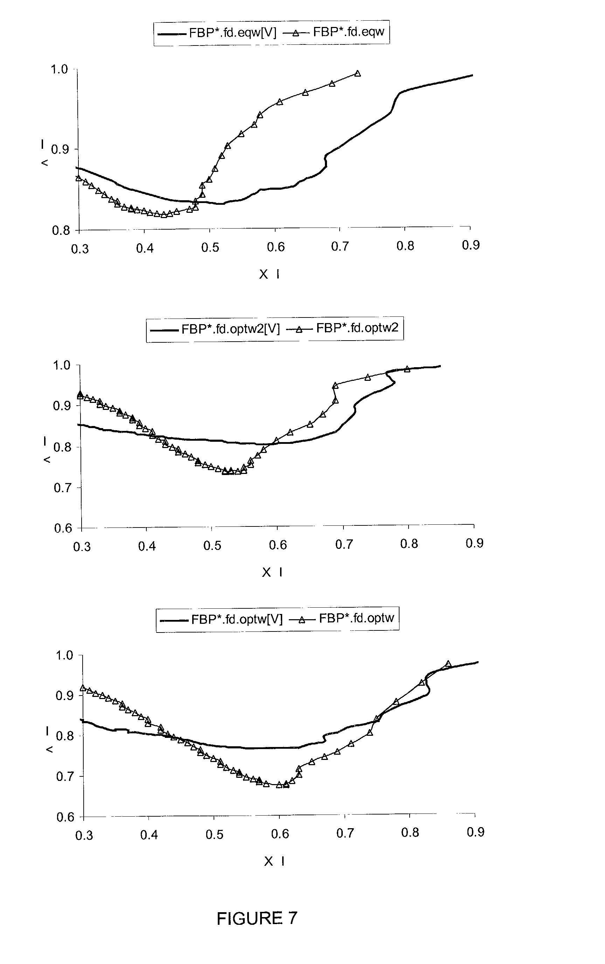

The relative performances of three FBPA metrics, -not optimized, partially optimized and thoroughly optimized-, submitted to this validation test are compared to the ones they scored against the initial NB problem in Figure 7. FBP*.fd.eqw, not resulting from an optimization with respect to the initial data set, is seen to perform better against the validation problem. The overall optimality level of the "suboptimal" metric FBP*.fd.optw2 is, in the validation test, superior at very high consistency levels, while in the training environment it reaches a deeper minimum, but at slightly worse consistency. FBP*.fd.optw shows a more effective NB with respect to its training environment, this metric being indeed "specific" to the given configuration of targets in the panel - and therefore a metric of choice in similarity screening searches for GPCR-binding active analogs.

The invention also relates, generally, to the use of a method as described above, for the production, selection, identification, design or optimization of a molecule. In particular, the invention relates to a method of producing a drug candidate, comprising (virtually) synthesizing a molecule and determining or predicting its activity by using a method as described above. An other object of this invention is a method of improving the biological activity or properties of a molecule, comprising (virtually) synthesizing analogs of said molecule and determining or predicting their activity by using a method as described above. The invention also relates, generally, to any molecule produced, selected, designed or improved by a method as described above, as well as analogs thereof and pharmaceutical compositions comprising the same. The invention is suited in various areas such as medicine (anti-tumor, CNS, cardiovascular, infectious, metabolic diseases, etc.), agrochemicals, etc.

**Claims**

1. Method of predicting biological activity profiles of compounds against an entire panel of targets covering different therapeutic areas, wherein experimental activity profiles define a multidimensional activity space **characterizing** a molecule in terms of its biological properties, in which chemical compounds are represented by activity profile vectors given by their experimental responses in each of included tests, method **characterized by** estimating the location of a novel compound within the Activity Space by Neighborhood Behavior principle on the basis of its calculated location in a Structural Space that optimally maps onto the activity space, considering that the In Silico molecular dissimilarity metrics that apply to the chosen structural space have been calibrated in order to optimize this structure-to-activity mapping by achieving a best compromise between two quantitative NB criteria: consistency, to specifically avoid predicting as similar pairs of compounds with different activities, and completeness, to retrieve a maximum of similar compound pairs with similar activity profiles.

2. Method of prediction according to claim 1, wherein quantitative NB criteria of In Silico metrics are defined on the basis of profile and/or HTP data of standardized and comparable activity information for a series of compounds with respect to an entire battery of targets.

3. Method of prediction according to claim 2, wherein the consistency criterion captures the proficiency of a structural similarity metric to selectively perceive pairs of activity-related compounds as structurally similar, a measure of consistency $\sigma(\delta)$ being defined by comparing the average of the activity similarity score $<DA(m,M)>|_{P(\delta)}$ over a pair subset $P(\delta)$ with structural dissimilarity lower than $\delta$ with, at one end, the average over a randomly picked subset of the same size $N_{P(\delta)}$, and , at the other, $<DA(m,M)>_{O(\delta)}$, where $O(\delta)$ denotes a subset of the same size as $P(\delta)$ containing the $N_{P(\delta)}$ most similar pairs in activity space itself.

4. Method of prediction according to any preceding claim, wherein NB criteria are used as objective quality scores for the choice of the optimal functional form and fittable parameters of In Silico metrics based on a representation of molecular structures in a structural space by vectors of molecular descriptors.

5. Method of prediction according to claim 4, wherein the molecular structures are represented by the Fuzzy Bipolar Pharmacophore Autocorrelograms (FBPA).

6. Method of prediction according to any preceding claim, wherein a validation assessing the NB parameters of the optimal FBPA metrics against different compounds in a completely different activity space is considered in order to cross-check the extent to which the optimal parameters of this metric are biased by the actual choice of targets in the panel used for calibration.

7. Method of prediction according to claim 6, wherein the use of customized activity profiles defines "privileged" activity spaces for the training of "focused" In Silico similarity metrics with a maximal discriminative power with respect to characteristic ligands for the classes of receptors entered in the profile.

8. Method of prediction according to any preceding claim, wherein the use of activity profiles featuring a large and diverse set of biological targets defines a consensus activity space for the training of general-purpose In Silico similarity metric, potentially applicable to any other targets not included in the training activity space.

9. Method of prediction according to any preceding claim, wherein AN normalized FBPA descriptors are used in SS with fittable weighing pharmacophoric features and fuzziness parameter $(w_k, \alpha)$ to reduce the afflux of "false similar" pairs and therefore allow a wider selection of "true similars" at comparable consistency, the resulting metrics reaching an optimal tradeoff between consistency and completeness.

10. Method of prediction according to claim 2, wherein an overall optimality criterion is defined by comparing the weighted sum $\kappa N_{FS} + N_{PFD}$ of the number of $N_{FS}$ "falsely similar" pairs (compound pairs with an In Silico dissimilarity below a given threshold $\delta$, but In Vitro activity dissimilarity above a given threshold d) and the number of $N_{PFD}$ "potentially false dissimilar" pairs (compound pairs with an In Silico dissimilarity above $\delta$, but In Vitro activity dissimilarity below d), to the value of this sum that would have been achieved by a random selection of an equal size compound pair set instead of the selection based on the actual In Silico dissimilarity scores.

11. Method of prediction according to claim 10, wherein the In Vitro dissimilarity scores DA(m,M) are defined as a fuzzy measure of the effective number of targets with respect to which two compounds display a significant difference in affinity, the said significant difference in activity being determined in function of the difference of the experimental percentages of inhibition achieved by m and M in that biological test, at a concentration of 10μM.

12. Method of prediction according to claim 11, wherein the said effective number of targets with respect to which two compounds display a significant difference in affinity is scaled on the basis of inter-target correlation coefficients in order to account for the potential relatedness of different targets included in the activity profile.

13. Method of predicting an unknown biological property profile of an untested or unsynthesized molecule M, wherein, a calibrated metric of optimal NB being given according to any preceding claim, the prediction is performed by interpolating the corresponding properties of its nearest neighbors, from a reference set of compounds for which the measurement of the property profile of interest had been previously undertaken.

**14.** Method of prediction according to any one of the preceding claims, wherein a variable number of all the near neighbors within a given dissimilarity radius $\Delta S(m,M)<\delta$ are used in calculation, and a weighted averaging of the neighbor properties is introduced, with closer neighbors having larger weights, based on the NB principle, the weight $W(m)$ of each neighbor m is assumed to decrease exponentially with the considered In Silico dissimilarity score $\Delta S(m,M)$.

**15.** Method of prediction according to any one of the preceding claims, wherein an "Effective Number of Neighbors" (EFN) of the molecule M is defined $\Sigma W(m)$, neighbors that are further away from the molecule M count less than one "effective" neighbor.

**16.** Method of prediction according to any one of the preceding claims, wherein, the effective weighted spread of the $P(m)$ values ($RMS_W$) is a measure of homogeneity in the neighborhood of M, expectedly lower than the overall spread $RMS_P$ of that property over the entire set of compounds and.

**17.** Method of prediction according to any one of the preceding claims, wherein an overall confidence score is defined as the product of EFN and $((RMS_P-RMS_W)/RMS_P)$, such a confident score being maximal when the molecule M has many close neighbors and all these neighbors consistently displaying close values of the property P.

**18.** Use of a method according to any one of the preceding claims, for the production, selection, identification, design or optimization of a molecule.

FIGURE 1

FIGURE 2

FIGURE 3

FIGURE 4

FIGURE 5

FIGURE 6

FIGURE 7

# EP 1 302 866 A1

**European Patent Office**

## EUROPEAN SEARCH REPORT

Application Number

EP 01 40 2656

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | US 6 240 374 B1 (PATTERSON DAVID E ET AL) 29 May 2001 (2001-05-29) * column 1, line 1 - column 7, line 42 * * column 10, line 32 - line 67 * * column 15, line 25 - column 17, line 27; claim 3 * | 1-18 | G06F17/30 G06F17/50 |
| A | DREWRY D H ET AL: "Approaches to the design of combinatorial libraries" CHEMOMETRICS AND INTELLIGEMT LABORATORY SYSTEMS, ELSEVIER SCIENCE PUBLISHERS, AMSTERDAM, NL, vol. 48, no. 1, 14 June 1999 (1999-06-14), pages 1-20, XP004167956 ISSN: 0169-7439 * page 4, right-hand column, line 13, paragraph 3 - page 5, right-hand column, line 23 * * page 7, right-hand column, line 14 - line 41 * * page 8, left-hand column, line 21 - page 9, right-hand column, line 12 * * page 10, right-hand column, paragraph 6 - paragraph 6.1 * | 1-18 | |

TECHNICAL FIELDS SEARCHED (Int.Cl.7)

G06F

The present search report has been drawn up for all claims

| Place of search | Date of completion of the search | Examiner |
|---|---|---|
| THE HAGUE | 30 September 2002 | Fournier, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

19

**EP 1 302 866 A1**

**ANNEX TO THE EUROPEAN SEARCH REPORT
ON EUROPEAN PATENT APPLICATION NO.**

EP 01 40 2656

This annex lists the patent family members relating to the patent documents cited in the above-mentioned European search report.
The members are as contained in the European Patent Office EDP file on
The European Patent Office is in no way liable for these particulars which are merely given for the purpose of information.

30-09-2002

| Patent document cited in search report | | Publication date | Patent family member(s) | | Publication date |
|---|---|---|---|---|---|
| US 6240374 | B1 | 29-05-2001 | US | 6185506 B1 | 06-02-2001 |
| | | | US | 2002099525 A1 | 25-07-2002 |
| | | | US | 2002099526 A1 | 25-07-2002 |
| | | | AU | 1847997 A | 20-08-1997 |
| | | | EP | 0892963 A1 | 27-01-1999 |
| | | | WO | 9727559 A1 | 31-07-1997 |

EPO FORM P0459

For more details about this annex : see Official Journal of the European Patent Office, No. 12/82

20